# EUROPEAN PATENT SPECIFICATION

(11) **EP 1 626 620 B1**
(45) Date of publication and mention of the grant of the patent: **21.04.2010**
(21) Application number: 04734541.8
(22) Date of filing: 24.05.2004
(51) Int. Cl.: A01G 7/04, A01G 9/00

(54) **METHODS FOR ALTERING THE LEVEL OF PHYTOCHEMICALS IN PLANT CELLS BY APPLYING WAVELENGTHS OF LIGHT FROM 400 NM TO 700 NM AND APPARATUS THEREFORE**
METHODEN ZUR ÄNDERUNG DES GEHALTS AN PHYTOCHECHEMISCHEN VERBINDUNGEN IN PFLANZENZELLEN UMFASSEND DIE ANWENDUNG VON LICHT DER WELLENLÄNGE VON 400-700 NM SOWIE DAZUGEHÖRENDE APPARAT
PROCEDES D'ALTERATION DU NIVEAU DE SUBSTANCES PHYTOCHIMIQUES DANS DES CELLULES VEGETALES PAR APPLICATION DE LONGUEURS D'ONDES COMPRISES ENTRE 400 NM ET 700 NM ET APPAREIL DESTINE A CET EFFET

(30) Priority: 23.05.2003 GB 0311953; 23.05.2003 GB 0311954
(43) Date of publication of application: 22.02.2006
(60) Divisional application: 09006306.6
(73) Proprietor: Fotofresh Limited, Douglas Cork (IE)
(72) Inventor: Karpinski, Stanislaw, 14733 Tumba (SE)
(74) Representative: Wise, Stephen James
(86) International application number: PCT/GB2004/002211
(87) International publication number: WO 2004/103060

(56) References cited:
- FR-A- 2 542 567
- US-A- 4 817 332
- US-A- 5 299 383
- HIRATA KAZUMASA ET AL: "Stimulation of dimeric alkaloid production by near-ultraviolet light in multiple shoot cultures of Catharanthus roseus" JOURNAL OF FERMENTATION AND BIOENGINEERING, vol. 74, no. 4, 1992, pages 222-225, XP002291374 ISSN: 0922-338X
- KERCKHOFFS L H J ET AL: "Phytochrome control of anthocyanin biosynthesis in tomato seedlings: Analysis using photomorphogenic mutants" PHOTOCHEMISTRY AND PHOTOBIOLOGY, vol. 65, no. 2, 1997, pages 374-381, XP002291373 ISSN: 0031-8655

## Description

The present invention relates to a method for altering the level of phytochemicals in plant cells and/or plant tissue and means therefor. In particular, the invention relates to a method for altering the level of phytochemicals such as plant secondary metabolites in harvested plant cells and/or plant tissue by applying wavelengths of light thereto selected from the white light or visible spectrum and means therefor.

It is known that the application of light from the UV spectrum, such as UV-B and UV-C can help to increase the levels of for example 'essential oils' and secondary metabolites in whole plants. However, UV-B and UV-C is problematic to handle for humans and is heavily implicated in cancerous disease processes. As such, UV-B and UV-C light is considered potentially harmful to healthy mammalian tissue and is considered hazardous to use.

'Essential oils' are responsible in large part for the aromaticity associated with many plants, such as plants comprising perfumed flowers and herbs, such as culinary herbs. Essential oils consist mainly of terpenoids and can include such compounds as 1,8-cineole, limonene, linalool and β-ocimene. Other compounds which may be found in essential oils, that is, oils which are not terpenoids, can include phenyl-propanoid-derived compounds such as methyl chavicol, methyl cinnamate, eugenol, and methyl eugenol. Thus, the term essential oils' is used in a qualitative sense to encompass compounds as indicated herein which contribute to the aromaticity of plants such as perfumed ornamentals and culinary herbs.

Ultraviolet light (and specifically UV-B) is known to have effects on the levels of secondary compounds of the phenyl-propanoid pathway of plants via action on key regulatory enzymes such as phenylalaline ammonia-lyase (Kuhn, D.N. et al (1984) Proc. Natl. Acad. Sci., USA, 81, 1102-1106) and chalcone synthase (Batschauer, A. et al (1996) The Plant Journal 9, 63-69 and Christie, J.M. and Jenkins, G.I. (1996) The Plant Cell 8, 1555-1567). There are many published reports of UV-B stimulation of phenolic compounds, including surface flavonols and flavonoids (Cuadra, P. and Harborne, J.B. (1996) Zeitschrift für Faturforschung 51c, 671-680 and Cuadra, P. et al (1997) Phytochemistry 45, 1377-1383), anthocyanins (Yatsuhashi, H. et al (1982) Plant Physiology 70, 735-741 and Oelmüller, R. and Mohr, H. (1985). Proc. Natl. Acad. Sci., USA 82, 6124-6128) and betacyanins (Rudat, A. and Goring, H. (1995). J. Expl. Bot. 46, 129-134) and these compounds have been implicated both in plant defence (Chappell, J. and Hahlbrock, K. (1984) Nature 311, 76-78 and Guevara, P. et al (1997) Phyton 60, 137-140) and as protection against UV-light (Lois, R. (1994) Planta 194, 498-503; Ziska, L.H. et al (1992) Am. Jnl. Bot. 79, 863-871 and Fiusello, N. et al (1985) Allionia (Turin) 26, 79-88).

Although observations have been reported on the effects of certain bands of UV light and of infrared light in altering, typically increasing the levels of certain phytochemicals within plant cells, the available art appears to be silent on the effect of irradiating plant cells or tissue with light of other wavelengths.

One apparent exception is International Patent Specification WO 00/41557, which discloses a method of inducing or promoting anthocyanin formation in plants and/or fruit, more specifically red coloration in green apple varieties, by irradiation with UV-B light, a mixture of UV-B and white light, or a mixture of white and blue light. This procedure entails irradiation for a lengthy period of 6 hours to several days in order to achieve the intended result and indeed, in the case of irradiation with a mixture of white and blue light, no less than seven days. Accordingly, the method as a whole, and certainly in the case of irradiation solely with visible wavelength light, is economically viable only in the context of bulk product treatment on an industrial scale.

A recognised problem associated with harvested vegetables or harvested vegetable parts are that the levels of plant phytochemicals, such as plant secondary metabolites, starts to decrease post-harvest almost immediately. For example, as harvested vegetables are processed for freezing and/or canning or are simply placed in refrigerators, such as domestic appliances or simply on open surfaces in a room for short periods for eating later by consumers, they lose much of their nutritional content in terms of the levels of phytochemicals found therein. The term "phytochemical" encompasses any chemical compound such as a secondary plant metabolite and which may be found naturally occurring in a plant. Such phytochemicals include antioxidants such as vitamins, e.g. vitamins C and/or E, glucosinolates, such as sinigrin, sulphoraphane, 4-methylsulphinylbutyl glucosinolate, and/or 3 methyl - sulphinylpropyl glucosinolate, progoitrin and glucobrassicin, isothiocyanates, indoles (products of glucosinolate hydrolysis), glutathione, carotenoids such as beta-carotene, lycopene, and the xanthophyll carotenoids such as lutein and zeaxanthin, phenolics comprising the flavonoids such as the flavonols (e.g. quercetin, rutin), the flavans/tannins (such as the procyanidins comprising coumarin, proanthocyanidins, catechins, and anthocyanins), flavones (e.g luteolin from artichokes), phytoestrogens such as coumestans, lignans, resveratrol, isoflavones e.g. genistein, daidzein, and glycitein, and resorcyclic acid lactones, and organosulphur compounds, phytosterols, terpenoids such as carnosol, rosmarinic acid, glycyrrhizin and saponins, and chlorophyll and chlorphyllin, sugars, and other food products such as anthocyanins, vanilla and other fruit and vegetable flavours and texture modifying agents and the like. Research indicates that the antioxidant properties of certain phytochemicals may help protect against the effects of ageing and chronic diseases, such as cancer and cardiovascular disease in mammals, and in particular in humans.

Phytochemicals can also serve as pharmaceutical compounds per se in mammalian species, such as humans, or pharmaceutically active derivatives can be synthesised from other intermediate compounds therefore that are found in plants and may be isolated therefrom. Thus, "phytochemicals" that may be substantially pharmaceutically inactive may find a use in providing or in being intermediates for the synthesis of active agents for the treatment of diseases such as cancers, and/or in pain management of mammals suffering from diseases, such as humans. Thus, plant chemicals falling under the definition of "phytochemicals" herein and that are known to be useful in the design of and/or provision of pharmaceutically active compounds include vincristine and vinblastine from *Catharanthus roseus,* taxanes such as those described in USP5665576, for example, taxol (paclitaxel), baccatin III, 10-desacetylbaccatin III, 10-desacetyl taxol, xylosyl taxol, 7-epitaxol, 7-epibaccatin III, 10-desacetylcephalomannine, 7-epicephalomannine, taxotere, cephalomannine, xylosyl cephalomannine, taxagifine, 8-benxoyloxy taxagifine, 9-acetyloxy taxusin, 9-hydroxy taxusin, taiwanxam, taxane Ia, taxane Ib, taxane Ic, taxane Id, GMP paclitaxel, 9-dihydro 13-acetylbaccatin III, and 10-desacetyl-7-epitaxol from plants of the family Taxaceae such as plants of the genera Amentotaxus, Austrotaxus, Pseudotaxus, Torreya and Taxus, for example from plants of the genus Taxus, such as T. brevifolia, T. baccata, T. x media (e.g. Taxus media hicksii, Taxus x media Rehder), T. wallichiana, T. Canadensis, T. cuspidata, T. floridiana, T. celebica, and T. x hunnewelliana, T. Canadensis, and tetrahydrocannabinol (THC) and cannabidiol (CBD) from cannabis plants such as *Cannabis sativa, Cannabis indica*, and *Cannabis ruderalis*, and other pharmaceuticals such as genistein, diadzein, codeine, morphine, quinine, shikonin, ajmalacine, serpentine and the like.

It has now been observed that by exposing or directing certain wavelengths selected from those making up white light onto harvested plant material such as green plant parts or plant cells comprising chlorophyll the level of phytochemicals therein can be transiently increased. Such phytochemicals include secondary metabolites as described herein and other phytochemicals for use as pharmaceuticals as alluded to herein. As a consequence, the level of desired plant phytochemicals, such as plant secondary metabolites e.g. antioxidants, can be increased in harvested plant material by the simple application of wavelengths of light for relatively short periods of time selected from those wavelengths or bands found in cold light, that is, visible light.

According to the present invention there is provided a method of transiently altering the level of at least one phytochemical selected from antioxidants in a harvested plant cell comprising chlorophyll or in harvested plant tissue comprising chlorophyll, the plant cell or tissue being capable of photosynthesis, by irradiating the said plant cell or plant tissue with white light enriched with blue light of at least one wavelength selected from the range of wavelengths of from 420nm to 490nm to induce or saturate oxygen evolution at the photosystem II reaction centre, wherein the irradiation is carried out over a time interval of up to 180 minutes.

The wavelength of light used may be of a single wavelength or a combination of at least two selected wavelengths within the range of from 420nm to 450nm such that it or they are capable of altering the level of said at least one phytochemical found in a plant cell or in plant tissue, typically raising the level upon exposure over a suitable time interval and at a suitable light intensity. Thus, the skilled addressee will appreciate that the wavelengths of light used in the present invention on plant material such as harvested vegetables or green leaf matter or green plant cells in culture, such as moss cells eg cells of *physcomitrella patens*, according to the method of the invention do not constitute all of the wavelengths of light making up white light, but a selection of them. Furthermore, it is to be understood that the light wavelength or wavelengths employed in the present invention are selected from so-called 'cold light' wavelengths, that is, the light used in the present invention does not comprise UV wavelengths and does not constitute infrared wavelengths, both forms of which are potentially hazardous to use. In a preferred embodiment, the wavelength or band of light used is white light enriched with blue light and red light and lies in the range of from 420nm to 700nm, preferably from 450nm to 700nm, depending on design and the phytochemical of interest. A suitable set of wavelengths that has been found to influence the level of certain phytochemicals in plant tissue is from 420nm - 700 nm with a capacity of up to 2000 microM/m²/s⁻¹ from above that is to the ventral side of the leaf, up to 700nm from the lateral (side) and dorsal (underside) side, e.g. from 650nm - 700nm with a capacity of 600 microM/m⁻²/s⁻¹, or any combination of two or three wavelengths thereof for a period up to 180 mins depending on design, the light intensity and plant material used. It has now been found that white light enriched with blue light and optionally also with red light appears to be particularly suitable for altering the level of phytochemicals within plant tissue comprised of a plant cell or plant cells that is/are capable of photosynthesis. The red wavelength may be selected from a wavelength within the range of from 600nm - 700nm, preferably from 620nm - 690 nm, more preferably from 625nm - 680 nm, and generally at about 650nm +/- 15nm. The wavelength of blue light is typically selected from a wavelength within the range of from 420nm - 490nm, preferably from 430nm - 470nm, more preferably from 435nm - 465nm and generally at about 450nm +/- 15nm. A combination of both red and blue light at any given energy ratio may be employed in the method of the invention. For instance, the energy ratio of Blue light:Red light may be selected from within the range of from 7:1 to 1:7, 6:1 to 1:6, such as 5:1 to 1:5, such as 5:2 to 2:5, 5:3 to 3:5, or 5:4 to 4:5. Other Blue light: Red light ratios may be selected from within the ranges 4:1 to 1:4, 3:1 to 1:3, 2:1 to 1:2, and 1:1 and any permutation within these ranges depending on design. The actual ratio selected may depend on species, age of plant parts, the phytochemical of interest and design. Typically, one unit of energy for blue light is about 50 - 150 microM/m⁻²/s⁻¹ +/- 30 microM/m⁻²/s⁻¹, for example 100 microM/m⁻²/s⁻¹ +/- 30 MicroM/m⁻²/s⁻¹. Typically, one unit of energy for red light is about 50 - 100 microM/m⁻²/ s⁻¹ +/- 10 microM/m⁻²/ s⁻¹, for example,75 microM/m⁻²/ s⁻¹ +/- 10 microM/m⁻²/s⁻¹. From such values or approximations the light intensity of blue and red light used together in a blue light:red light ratio shone onto plant material such as leaf surfaces may be calculated. Naturally, the skilled addressee will appreciate that depending on the plant cells or plant tissue employed, the length of time that the plant cells or tissue is exposed to light of wavelengths outlined herein will alter with design. The length of time that plant cells or plant tissue may be exposed to wavelengths used in the present invention for an effect on phytochemical levels to be observed lies in the range up to 180 minutes. Preferably, the exposure is up to 100 minutes. More preferably, the exposure is up to 60 minutes, and preferably still up to 45 minutes. Yet more preferably, the exposure is up to 30 minutes and more preferably again, from 5 to 15 minutes. Typically, the level of phytochemicals is elevated on the application of light to the plant tissue or plant cell culture over short time intervals as alluded to herein.

The invention can be employed on any plant tissue that is capable of responding to exposure to or irradiation with wavelengths of light as outlined herein. The plant tissue comprises tissue that is capable of photosynthesis. Plant material that can be used in the method of the invention includes all green vegetables and green seeds, e.g. peas, green beans, spinach, species from the *Brassica oleracea* such as broccoli, green cabbage, red cabbage, Brussels sprouts, kohlrabi, cauliflower, white cabbage, and the like, and all plant material, such as green plant material, for example, cells comprising chlorophyll, green stems, calyx, leaves, and the like that is able to respond to wavelengths of light selected from the range 400nm to 700nm as hereindescribed. Other plant material that may be treated in accordance with methods of the invention may be green material such as green needles derived from non-vegetable sources such as plants of the order Taxaceae as described herein, tea leaves, and of cells grown in plant cell cultures in bioreactors such as moss cells and tissues (e.g. protonema) from physcomitrella patens, and other plant cell cultures eg callus cell cultures, cultures of lemnospora species, algae or even somatic embryo clusters.

In a method not exemplifying the invention, the phytochemical content in live plant cells or plant tissue in an environment is raised by exposing the said plant cells or tissue with light of at least a wavelength selected from light of wavelengths found in cold light from an artificial light source. Naturally, the skilled addressee will appreciate that white light enriched with the selected wavelength(s) of light as described herein that alters the phytochemical profile of a plant cell or plant tissue, such as a harvested tissue lies within the ambit of the invention. In a preferred embodiment, the plant tissue of interest is exposed to light consisting of the wavelengths selected from the range of wavelengths 420nm - 700nm, such as a combination of white light and red light, red light per se, a combination of red and blue light, red, blue and white light, blue light or white light enriched with blue light and the like. Preferably, the combination of light sources includes red light of a wavelength that may be selected from a wavelength within the range of from 600nm - 700nm, preferably from 620nm - 690 nm, more preferably from 625nm - 680 nm, and generally at about 650nm +/- 15nm. The wavelength of blue light is typically selected from a wavelength within the range of from 420nm - 490nm, preferably from 430nm - 470nm, more preferably from 435nm - 465nm and generally at about 450nm +/- 15nm. Red or blue light or a combination of both red and blue light or a combination of red and/or blue light with white light at any selected energy ratio as hereindescribed may be employed in the method of the invention. The said plant cells or plant tissue can be located under cover. 'Under cover' means that the cells or tissue is located under cover when exposed, for example, during a food processing step prior to further processing such as freezing or canning or heat treating or cooking as alluded to hereinbelow.

'Cover' is to be understood as a general term and may be taken to mean a receptacle in which the plant material or plant cells may be placed, for example a closed container with a built-in light source therein, such as a refrigerator unit comprising an inbuilt light source that can be activated on demand for a pre-determined time interval. Thus, use can be made of a cooling means, such as a conventional refrigerator comprising a light source capable of emitting light selected from the wavelengths of light as hereindescribed. Alternatively, 'under cover' may be taken to mean a processing factory wherein harvested plant material is exposed to one or more light sources producing light of appropriate wavelength or wavelengths over a short period of time during the processing operation, such as canning, freezing plant material, or immediately prior to the cooking of foods for canning or for baby food manufacture eg purees and the like, and further processed foods such as soups, vegetable-based sauces and the like.

"Plant cells" also includes those plant parts or tissues which display an aromaticity due to the presence of volatile phytochemicals, which for the purposes of the present invention are included in the definition of "phytochemicals" herein, and which is detectable by the human olfactory senses when such plant cells making up a plant part are cut or harvested. Such plants may display the aromaticity naturally, for example in the case of cut herbs, from the cut leaves. The plant cells or tissue or parts include members of the Labiatae, such as the broad-leafed herbs. Suitable examples of broad-leafed herbs include basil, oregano, sage, coriander, dill, marjoram and thyme. Other herbs, such as cut herbs that may benefit from being treated according to the present invention include chives, garlic, bay leaf, lemon balm, mint, lavender, parsley, the fennels, eg bronze fennel and common fennel, and the like. A more complete list of common herbs to which the invention can be applied is to be found in Taylors Guide to Herbs 1995, Eds. Buchanan R. & Tenebaum F. Houghton Mifflin Co. New York. Naturally, the skilled addressee will appreciate that the said plant cells or plant parts are alive when exposed to light in accordance with the present invention and are capable of responding to the application of the cold light- derived light stimulus.

Plant cells or plant parts may be harvested at any stage of growth so long as the harvested plant cells or tissue are capable of responding to the application of light of wavelength and duration as outlined herein. In a preferred embodiment, the harvested plant cells or tissue of broad - leaf herbs can be exposed to wavelengths of light used in the present invention from the 3 to 4 leaf stage and most preferably in the case of culinary herbs such as basil, the 5-leaf stage. It is envisaged that plant cells and/or tissue such as culinary herbs and green vegetables are most usefully exposed as herein-described immediately before processing (e.g. freeze drying, adding to processed foods such as sauces, soups, canned goods and the like), that is to say after the harvesting of cuttings from such plants and/or the provision of young plants for processing e.g. as dried herbs. Dried herbs treated with light as outlined herein immediately post-harvest, for a short period of time, particularly those measured at the 5-leaf stage, are considered to display an increased aromaticity relative to controls which are not exposed to light as described herein.

The artificial light source can be of any suitable conventional source, such as a light emitting diode or even a white light source comprising filters that let through light of the desired wavelength(s). The light source may be placed at any distance from the harvested material provided that the light energy used is sufficient to influence, for example to induce or saturate oxygen evolution at the photosystem II reaction centre and/or to trigger, that is set off, a transient photo-oxidative stress and/or a moderate photosynthetic electron transport inhibition. Optimising of the light energy and light composition may be performed for example, by monitoring oxygen evolution and chlorophyll fluorescence using conventional methods (e.g. according to the instruction manual and software of Hansatech Instruments Ltd., King's Lynn, UK). It is preferable to locate the light source in a position which affords the greatest amount of irradiation per square unit (e.g. cm², m² etc.) of the harvested plant material. Suitably, depending on the size of the covered area, for example that of a processing compartment in a processing factory, or of a fridge or other container such as a microwave oven or magnetron fitted with a suitable light source capable of being manually or automatically activated, for example, by employing a timing means and thereby emitting wavelengths of light as indicated herein and described herein. Alternatively, an independent container specifically designed for exposing plant parts or cells to light of wavelengths as described herein may be employed. In a further alternative, the number of light sources may be as little as one to a whole 'battery' of light sources arranged in series and/or in parallel, for example, in a food processing factory setting, each light source being suitably distanced one from the other at appropriate intervals in such a manner as to effect exposure of the plant material to light of wavelengths as described herein which results in a significant alteration in the level of phytochemicals found therein, preferably an increase of desired phytochemicals.

Apparatus for performance of the method in accordance with any of the preceding aspects may comprise an enclosure defining an exposure chamber, support means disposed in the chamber for supporting plant material therein in such a manner and position as to permit exposure to light from a plurality of directions, and light generating and applying means to generate light of at least one predetermined wavelength and to apply the generated light to the supported plant material for a predetermined period of time and from a plurality of directions to provide exposure of the material to the light from more than one side.

The enclosure preferably has the form of a housing of any suitable volumetric form, for example cuboidal, which is closed at at least some of its sides. Such a housing can range from a relatively small-size benchtop appliance of the kind compatible with domestic use, for example similar in concept to a microwave oven, through medium-size equipment suitable for use in commercial food preparation premises, for example a restaurant, to a large-size installation appropriate to bulk material treatment in an industrial context, such as a food-processing plant. In the case of larger size applications, the enclosure may take the form of a structure bounded by walls, a base and a ceiling, at least some of which are provided by integral or fitted internal elements of a building.

The exposure chamber defined by the enclosure can similarly be of any appropriate volume, but preferably is so dimensioned that the lengths of the light paths in at least the majority of the directions enable exposure of the material to a predetermined intensity of light for a given minimum expenditure of operating energy of the light generating and applying means. Thus, the enclosure can be large enough to accommodate light paths to the supported plant material in all the intended directions, but preferably not so large that the paths are of such length that an undue expenditure of energy is necessary to ensure application of the requisite intensity of light.

The support means is preferably disposed so that the light can reach several sides of the material for exposure thereof to the light over a predetermined minimum proportion of its area. In a basic form it can comprise a member, such as a shelf, forming a surface on which the plant material can be placed. The member in that case should be light-permeable, whether by use of transparent material such as glass or clear plastics or by construction from, for example, intrinsically non-transparent or opaque material having light passage openings, for example a grating, mesh or apertured plate. Other forms of support means are equally possible depending on the kind of plant material, for example strips engageable under end portions of the material if of stable form, clamps or clips to fix and stretch or suspend the material, a pin or pins to support the material punctiformly or even skewer the material or a receptacle - whether transparent or perforated - to receive the material, particularly loose material.

Moreover, the support means can be stationary or mobile depending on whether the plant material is to reside in the chamber in a fixed location or to move through the chamber. In the case of movement of the material, the support means can be stationary and the enclosure itself, inclusive of the light generating and applying means, can be mobile so as to travel, perhaps in reciprocating manner, relative to the support means and in the supported material. In the case of mobile support means or a mobile enclosure, the enclosure may be formed with one or more openings defining an entrance and exit or a combined exit/entrance, the or each opening being optionally closable by a door or other closure means.

The light generating and applying means preferably comprises a plurality of light sources to emit light in different directions, a single light source with a plurality of reflectors to reflect light from the source in different directions, or a plurality of light sources and a plurality of reflectors to emit light and reflect light, respectively, in different directions. The or each such light source can comprise, for example, a light-emitting diode. The light generating and applying means transmits light in the blue wavelength, in combination with white light. The use of reflectors reduces energy costs at the expense of some attenuation of the light intensity, which may or may not be of consequence, depending on the size of the exposure chamber and quantity of plant material to be treated. The number and disposition of the light source or light sources and reflectors is thus preferably selected in dependence on constructional parameters of the apparatus and also parameters of the particular method of treatment. For preference, the light emitting and/or light reflecting components of the light generating and applying means are disposed at a plurality of sides of the enclosure. In a small-size appliance, the light sources may, for convenience in the provision of the power supply, be mounted in the same general region, for example a ceiling of the chamber, and reflectors provided in the region of the base of the chamber. The light exit surfaces of the sources and the planes of reflective surfaces of the reflectors can be oriented to ensure that light of the selected wavelength or wavelengths is aimed directly at the top, bottom and sides of the supported plant material. Such light sources can be, apart from light-emitting diodes, single lamps or arrays of lamps, for example incandescent bulbs or fluorescent tubelights. The reflectors can be, for example, mirrors, polished metal panels or simply reflective coatings or coverings applied to appropriately oriented internal surfaces of the enclosure. Emission of light in the preferred wavelength range of 400 to 700 nm can be achieved by transmitting the light emitted by the or each source through a transmission filter passing on light only of a selected specific wavelength. Similarly the duration of application of the light to the supported plant material can be controlled by switching means to switch off the light generating and applying means, such as by switching off operating voltage of the light source or sources after operation for the predetermined period of time. Preferably the control is by way of timing means with a time selection facility to select the predetermined period of time. Control of duration of exposure to the light can, however, equally well be achieved by other optical measures including screening or shielding the plant material, screening or shielding the light source or sources and reflector or reflectors, and influencing selectably reflective surfaces to become light transmissive. Alternatively, the treated plant material can be removed from the exposure chamber at the conclusion of the predetermined time period, whether by ejection after a dwell time in a rest state or by departure from the chamber after travel therethrough for the predetermined period, such travel embracing both movement of the support means supporting the material and movement of the enclosure inclusive of light source or sources and any associated reflectors.

It is to be understood that the teaching of all references cited herein is incorporated into the instant specification.

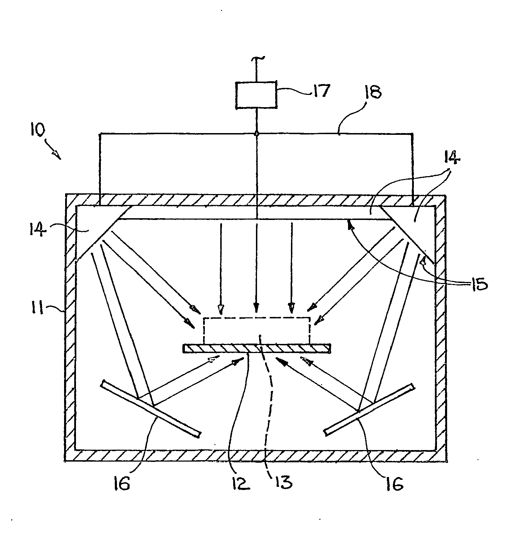

The invention will now be described with reference to the following examples and accompanying drawing (Figure 1). It is to be understood that the examples and information presented in Figure 1 are not to be viewed as limiting the scope of the invention in any way.

### EXPERIMENTAL

*Plant Material*. Cut Chinese cabbage (bokchoi), green broccoli, and spinach were obtained from a supermarket. Arabidopsis thaliana Col-0 plants were obtained from the Nottingham Arabidopsis stock centre.

*Light Treatments*. Plant parts/leaves were irradiated with light intensity of 1400 microM/m⁻²/s⁻¹ from above and 180 microM/m⁻²/s⁻¹ from below. Plant parts/leaves were kept on a transparent dish and sprayed with water and saturated with CO₂ which helped prevent the plant parts/leaves from drying out and supplied extra CO₂ for photosynthesis during light exposure.

1400 microM/m⁻²/s⁻¹: Light source consisted of two Futur LED red Type R210R2-MF, Swarco Austria (total of 180 microM/m⁻²/s⁻¹ of 680nm +/- 20nm); 300 microM/m⁻²/s⁻¹ of red filtered light (General Electric Quartzline EHJ, 250W 24V light) with a transmission filter of >650 and <700 nm; the rest (920 microM/m⁻²/s⁻¹) was white halogen light (General Electric Quartzline EHJ, 250W, 24V light).

180 microM/m⁻²/s⁻¹: Light source consisted of two Futur LED Red Type R210R2-M, Swarco Austria lights (total 180 microM/m⁻²/s⁻¹ of 680nm +/- 10 nm.

Environmental conditions: Temperature 20 degrees centigrade; humidity 80%.

*Analysis of Plant Material:* 20 - 50 grams of plant material from each plant type was used for exposure to different light intensities as described above. 3x 1 gram samples were used for analysis, and the analysis repeated three times.

**Measurement of Ascorbate Levels:** Yoshimura, K et al (2000) Plant Physiol. 123, 223 - 233

**Measurement of Glutathione Levels**: Karpinski, S. et al (1997) Plant Cell, 9, 627 - 642; Creissen G et al (1999) Plant Cell, 11, 1277-1291.

### Results

**Chin Cab.**

| µ**moles in extract** | **Tissue g FW** | **Tissue conc** µ**mol / g FW** | **mean conc** µ**mol / g FW** | **SD** |
|---|---|---|---|---|
| 0,116 | 0,200 | 0,58 | 0,49 | 0,08892681 |
| 0,114 | 0,250 | 0,46 | | |
| 0,103 | 0,190 | 0,54 | | |
| 0,118 | 0,200 | 0,59 | | |
| 0,171 | 0,340 | 0,50 | | |
| 0,087 | 0,220 | 0,40 | | |
| 0,120 | 0,320 | 0,37 | | |
| 0,099 | 0,170 | 0,58 | | |
| 0,078 | 0,200 | 0,39 | | |
| 0,236 | 0,200 | 1,18 | 1,20 | 0,16252455 |
| 0,333 | 0,250 | 1,33 | | |
| 0,256 | 0,190 | 1,35 | | |
| 0,198 | 0,200 | 0,99 | | |
| 0,317 | 0,340 | 0,93 | | |
| 0,310 | 0,220 | 1,41 | | |
| 0,363 | 0,320 | 1,13 | | |
| 0,222 | 0,170 | 1,31 | | |
| 0,240 | 0,200 | 1,20 | | |

**Broccoli**

| µ**moles in extract** | **Tissue g FW** | **Tissue conc** µ**mol / g FW** | **mean conc** µ**mol / g FW** | **SD** |
|---|---|---|---|---|
| 0,100 | 0,200 | 0,50 | 0,69 | 0,15479792 |
| 0,181 | 0,230 | 0,79 | | |
| 0,164 | 0,170 | 0,97 | | |
| 0,295 | 0,400 | 0,74 | | |
| 0,246 | 0,400 | 0,62 | | |
| 0,177 | 0,340 | 0,52 | | |
| 0,167 | 0,200 | 0,84 | | |
| 0,224 | 0,350 | 0,64 | | |
| 0,059 | 0,100 | 0,59 | | |
| 0,149 | 0,100 | 1,49 | 1,23 | 0,16203449 |
| 0,245 | 0,200 | 1,23 | | |
| 0,313 | 0,250 | 1,25 | | |
| 0,185 | 0,150 | 1,23 | | |
| 0,377 | 0,300 | 1,26 | | |
| 0,282 | 0,200 | 1,41 | | |
| 0,228 | 0,200 | 1,14 | | |
| 0,155 | 0,150 | 1,04 | | |
| 0,196 | 0,200 | 0,98 | | |

**Spinach**

| µ**moles in extract** | **Tissue g FW** | **Tissue conc** µ**mol / g FW** | **mean conc** µ**mol / g FW** | **SD** |
|---|---|---|---|---|
| 0,093 | 0,215 | 0,43 | 0,47 | 0,07651205 |
| 0,127 | 0,279 | 0,45 | | |
| 0,120 | 0,250 | 0,48 | | |
| 0,112 | 0,200 | 0,56 | | |
| 0,150 | 0,457 | 0,33 | | |
| 0,151 | 0,340 | 0,44 | | |
| 0,131 | 0,270 | 0,49 | | |
| 0,108 | 0,220 | 0,49 | | |
| 0,089 | 0,150 | 0,59 | | |
| 0,224 | 0,270 | 0,83 | 1,04 | 0,19301555 |
| 0,306 | 0,215 | 1,42 | | |
| 0,345 | 0,319 | 1,08 | | |
| 0,123 | 0,100 | 1,23 | | |
| 0,228 | 0,210 | 1,09 | | |
| 0,188 | 0,200 | 0,94 | | |
| 0,306 | 0,300 | 1,02 | | |
| 0,207 | 0,250 | 0,83 | | |
| 0,260 | 0,280 | 0,93 | | |

**Arabidopsis**

| µ**moles in extract** | **Tissue g FW** | **Tissue conc** µ**mol / g FW** | **mean conc** µ**mol / g FW** | **SD** |
|---|---|---|---|---|
| 0,193 | 0,150 | 1,29 | 1,03 | 0,17199289 |
| 0,215 | 0,200 | 1,07 | | |
| 0,119 | 0,100 | 1,19 | | |
| 0,102 | 0,100 | 1,02 | | |
| 0,084 | 0,100 | 0,84 | | |
| 0,219 | 0,200 | 1,09 | | |
| 0,108 | 0,100 | 1,08 | | |
| 0,110 | 0,150 | 0,73 | | |
| 0,091 | 0,100 | 0,91 | | |
| 0,197 | 0,090 | 2,19 | 2,40 | 0,40223793 |
| 0,282 | 0,100 | 2,82 | | |
| 0,376 | 0,150 | 2,51 | | |
| 0,337 | 0,150 | 2,25 | | |
| 0,415 | 0,150 | 2,77 | | |
| 0,282 | 0,100 | 2,82 | | |
| 0,312 | 0,150 | 2,08 | | |
| 0,243 | 0,150 | 1,62 | | |
| 0,252 | 0,100 | 2,52 | | |

Ascorbate content in micro-mol per gram FW before and after 90 min exposure in PLP.

| Chin.Cab | Broccoli | Spinach | Arabidopsis | |
|---|---|---|---|---|
| 1.2 ±0.16 | 0.7 ±0.15 | 0.47 ±0.07 | 2.4 | |
| 0.5 ±0.1 | 1.2 ±0.16 | 1.05 ±0.19 | 1.03 ±17 ±0.4 | |

**Chin. Cab.**

| Total GSH | Chin. Cab | | | | | |
|---|---|---|---|---|---|---|
| **Sample** | **Area** | **Amount nmoles/** | **nmoles g/FW** | | mean val. | SD |
| | 1 | 31654089 | 10,859 | 181,887 | 180,494 | 29,3206795 |
| | 3 | 41101872 | 13,447 | 225,244 | | |
| | 4 | 27238531 | 9,649 | 161,623 | | |
| | 5 | 27295469 | 9,665 | 161,885 | | |
| | 6 | 29161120 | 10,176 | 170,446 | | |
| | 7 | 36141765 | 12,088 | 202,482 | | |
| | 8 | 21379688 | 8,044 | 134,736 | | |
| | 9 | 36831972 | 12,277 | 205,649 | | |
| 1* | | 39296546 | 12,952 | 216,959 | 281,096 | 69,9886728 |
| 3* | | 43822014 | 14,192 | 237,728 | | |
| 4* | | 48533904 | 15,483 | 259,351 | | |
| 5* | | 34841563 | 11,732 | 196,515 | | |
| 6* | | 50240901 | 15,951 | 267,185 | | |
| 7* | | 69414709 | 21,204 | 355,176 | | |
| 8* | | 78703191 | 23,749 | 397,803 | | |
| 9* | | 61325543 | 18,988 | 318,054 | | |

**Broccoli**

| Total GSH | Broccoli | | | | | |
|---|---|---|---|---|---|---|
| **Sample** | **Area** | **Amount nmoles/** | **nmoles g/FW** | | mean val. | SD |
| | 1 | 41736924 | 13,621 | 228,159 | 232,174 | 31,9447456 |
| | 3 | 51946723 | 16,418 | 275,013 | | |
| | 4 | 48612523 | 15,505 | 259,712 | | |
| | 5 | 40125988 | 13,180 | 220,766 | | |
| | 6 | 39622245 | 13,042 | 218,454 | | |
| | 7 | 40026742 | 13,153 | 220,310 | | |
| | 8 | 30111456 | 10,436 | 174,808 | | |
| | 9 | 48711647 | 15,532 | 260,167 | | |
| 1* | | 96714429 | 28,683 | 480,459 | 405,250 | 67,6418849 |
| 3* | | 84627781 | 25,372 | 424,992 | | |
| 4* | | 66388725 | 20,375 | 341,290 | | |
| 5* | | 68466721 | 20,944 | 350,826 | | |
| 6* | | 99927747 | 29,564 | 495,205 | | |
| 7* | | 76245116 | 23,075 | 386,522 | | |
| 8* | | 60224542 | 18,686 | 313,001 | | |
| 9* | | 90013325 | 26,847 | 449,707 | | |

**Spinach**

| Total GSH | Spinach | | | | | |
|---|---|---|---|---|---|---|
| **Sample** | **Area** | **Amount nmoles/** | **nmoles g/FW** | | mean val. | SD |
| | 1 | 16715549 | 6,766 | 113,332 | 123,873 | 22,6697689 |
| | 3 | 19755348 | 7,599 | 127,282 | | |
| | 4 | 25672399 | 9,220 | 154,436 | | |
| | 5 | 24672953 | 8,946 | 149,849 | | |
| | 6 | 10044238 | 4,938 | 82,716 | | |
| | 7 | 16335627 | 6,662 | 111,588 | | |
| | 8 | 20004652 | 7,667 | 128,426 | | |
| | 9 | 18899746 | 7,364 | 123,355 | | |
| 1* | | 53397468 | 16,816 | 281,671 | 271,408 | 65,4267522 |
| 3* | | 58951541 | 18,337 | 307,159 | | |
| 4* | | 40128664 | 13,180 | 220,778 | | |
| 5* | | 37552495 | 12,475 | 208,956 | | |
| 6* | | 40002342 | 13,146 | 220,198 | | |
| 7* | | 42987689 | 13,964 | 233,899 | | |
| 8* | | 56244911 | 17,596 | 294,738 | | |
| 9* | | 80023341 | 24,111 | 403,861 | | |

**Arabidopsis**

| Total GSH | Arabidopsis | | | | | |
|---|---|---|---|---|---|---|
| **Sample** | **Area** | **Amount nmoles/** | **nmoles g/FW** | | mean val. | SD |
| | 1 | 11072453 | 5,220 | 87,435 | 97,860 | 19,2025604 |
| | 3 | 10999341 | 5,200 | 87,099 | | |
| | 4 | 15494428 | 6,431 | 107,728 | | |
| | 5 | 20598562 | 7,830 | 131,151 | | |
| | 6 | 9534478 | 4,798 | 80,377 | | |
| | 7 | 9387374 | 4,758 | 79,702 | | |
| | 8 | 11667236 | 5,383 | 90,164 | | |
| | 9 | 17999245 | 7,118 | 119,223 | | |
| 1* | | 41009363 | 13,422 | 224,820 | 215,027 | 41,0803789 |
| 3* | | 27701187 | 9,776 | 163,747 | | |
| 4* | | 25622298 | 9,206 | 154,206 | | |
| 5* | | 42888934 | 13,937 | 233,445 | | |
| 6* | | 36772672 | 12,261 | 205, 377 | | |
| 7* | | 50902445 | 16,132 | 270,221 | | |
| 8* | | 37772453 | 12,535 | 209,965 | | |
| 9* | | 48333874 | 15,428 | 258,433 | | |

Reduced glutathione content in nano-mol per gram FW before and after 90 min exposure in PLP.

| Chin Cab. | Broccoli | Spinach | Arabidopsis | |
|---|---|---|---|---|
| 180 ±29 | 232 ±32 | 123 ±22 | 97 ±19 | |
| 281 ±69 | 405 ±68 | 271 ±65 | 215 ±41 | |

### Peas - Vitamin C level

*Plant Material*. Fresh green Peas are obtained from a supermarket.

*Light Treatments*. Peas are irradiated with light intensity of 1400 microM/m⁻²/s⁻¹ from above and 180 microM/m⁻²/s⁻¹ from below.

1400 microM/m⁻²/s⁻¹: Light source consists of two Futur LED red Type R210R2-MF, Swarco Austria (total of 180 microM/m⁻²/s⁻¹ of 680nm +/- 20nm); 300 microM/m⁻²/s⁻¹ of red filtered light (General Electric Quartzline EHJ, 250W 24V light) with a transmission filter of >650 and <700 nm; the rest (920 microM/m⁻²/s⁻¹) is white halogen light (General Electric Quartzline EHJ, 250W, 24V light).

180 microM/m⁻²/s⁻¹: Light source consists of two Futur LED Red Type R210R2-M, Swarco Austria lights (total 180 microM/m⁻²/s⁻¹ of 680nm +/-10 nm.

Environmental conditions: Temperature 20 degrees centigrade; humidity 80%.

*Analysis of Plant Material:* Peas are exposed to different light intensities as described above. Treated peas are used for analysis. The vitamin content of control peas, that is, peas not subjected to the light treatment is also measured. Differences in vitamin C levels between control and treated peas (x3 replicates) are observed.

**Measurement of Ascorbate Levels:** Yoshimura, K et al (2000) Plant Physiol. 123, 223 - 233

### Brussels Sprouts, Green Cabbage - Vitamin C levels

*Plant Material.* Cut Brussels sprouts and green cabbage are obtained from a supermarket.

*Light Treatments.* Plant parts/ leaves are irradiated with light intensity of 1400 microM/m⁻²/s⁻¹ from above and 180 microM/m⁻²/s⁻¹ from below.

1400 microWm⁻²/s⁻¹: Light source consists of two Futur LED red Type R210R2-MF, Swarco Austria (total of 180 microM/m⁻²/s⁻¹ of 680nm +/- 20nm); 300 microM/m⁻²/s⁻¹ of red filtered light (General Electric Quartzline EHJ, 250W 24V light) with a transmission filter of >650 and <700 nm; the rest (920 microM/m⁻²/s⁻¹) is white halogen light (General Electric Quartzline EHJ, 250W, 24V light).

180 microM/m⁻²/s⁻¹: Light source consists of two Futur LED Red Type R210R2-M, Swarco Austria lights (total 180 microM/m⁻²/s⁻¹ of 680nm +/-10 nm.

Environmental conditions: Temperature 20 degrees centigrade; humidity 80%.

*Analysis of Plant Material:* 20 - 50 grams of plant material from each plant type is used for exposure to different light intensities as described above. 3 x 1gram samples are used for analysis, and the analysis repeated. Differences in vitamin C level between treated plant material and non-treated plant material (control) are observed.

**Measurement of Ascorbate Levels:** Yoshimura, K et al (2000) Plant Physiol. 123, 223 - 233

### Section 2: Exposure of Catharanthus roseus Leaves to Red and White Light and HPLC Analysis thereof.

### Introduction

HPLC analysis was carried out to determine the bisindole alkaloid content in *Catharanthus roseus* leaves. Such determinations were made to judge the effects of different treatments of C. *roseus* plants on alkaloid content in green material such as leaves.

The method used is that as described by D.A.C. Hallard et al (2000) PhD thesis: Transgenic Plant Cells for the Production of Indole Alkaloids, Leiden University.

### Methods

### Material

Plant growth conditions: *Catharanthus roseus* plants were grown in the greenhouse, under a long photoperiod (13 h) 200-400 microM/m⁻²/s⁻¹, temperature 22°C - day; 18°C night, and high relative humidity (70% +/- 5%). Leaves (top and adjacent leaves), from 15 week-old flowering *Catharanthus roseus* plants were detached and treated as described herein with the following modifications: Red light was shone on both the adaxial (top surface) and abaxial (bottom surface) of leaves at an intensity of 160 microM/m⁻²/s⁻¹ on each side for 3 hours; white light was shone onto the leaves for 2 hours.

| Sample No. | Fresh weight (mg) |
|---|---|
| (Internal designation) | |
| 01 Control in greenhouse | 370 |
| 02 Control in greenhouse | 290 |
| 03 Control in greenhouse | 350 |
| | |
| 24 Exposure to light first 2h (red + white) | 360 |
| 25 Exposure to light first 2h (red + white) | 230 |
| 26 Exposure to light first 2h (red + white) | 230 |
| | |
| R44 Exposure from 24-26 for one more h (red) | 360 |
| R45 Exposure from 24-26 for one more h (red) | 290 |
| R46 Exposure from 24-26 for one more h (red) | 490 |

### Extraction

Plant material was provided as lyophilised leaf material by Dr. S. Karpinski (Stockholm University, Sweden).

Each sample of material of approximately 10 mg was weighed accurately in triplicate (one sample - line 02 - in duplicate) and mixed thoroughly in Eppendorf cups with 0.50ml 0.1% Trifluoroacetic acid (TFA). Then the samples were sonicated for 30 minutes using a sonicator (sonicor, Copiague NY, USA), after which leaf debris was precipitated by centrifugation at 13000 rpm for 10 minutes. The supernatant was used for HPLC analysis. The samples were then collected and stored at -80°C for later analysis. A standard curve was made by using 4 dilutions of an equimolar mix of vincristine and vinblastine in the range 0.1 - 2 mM.

All fractions were analysed by HPLC with photodiode array (PDA) detection using a Waters 600E HPLC pump equipped with a 717 autosampler and a 990 photodiode array detector.

### HPLC

A Vydac 218MS54 column (250 × 4.6 mm lumen diameter), and a linear acetonitrile gradient in 0.01% TFA in water were used according to table 1. Injection volume was 50 µl.

**Table 1. Gradient used for HPLC Reverse Phase C-18 column (RP-18)**

| | | |
|---|---|---|
| 0-20 min: 1.00 ml/min | 15 - 23 % | CH₃CN |
| 20-30 min: 1.00 ml/min | 23 - 48 % | CH₃CN |
| 30-34 min: 1.00 ml/min | 48 % | CH₃CN |
| 34-35 min: 1.00 ml/min | 48 -15 % | CH₃CN |

Detection was achieved using a photodiode array detector in the wavelength range of 200 - 350 nm.

### Results & Discussion

### HPLC

From the HPLC chromatograms, the following results were obtained by integration of the peaks at a wavelength of 215 nm (see Table 2 for average values & Table 3 for the full set of results).

### Calibration curve

Both vincristine and vinblastine showed a linear response in the used range (although the highest value for vinblastine was very high and therefore not counted). Linear regression over 5 points (in a plot of area vs. nmoles injected) gave a correlation co-efficient of 0.041 and an r² of 0.9978.

### Samples

**Table 2. Concentrations in µmoles/g DW of some indole alkaloids in the leaf materials tested. Averages of triplicate determinations shown.**

| | **Ajmalicine** | **vindoline** | **vinblastine** | **?** | **AHVB** |
|---|---|---|---|---|---|
| **01** | 7.15 | 2.93 | 0.25 | 0.11 | 3.27 |
| **02** | 11.01 | 4.61 | 0.35 | 0.16 | 3.46 |
| **03** | 5.61 | 1.82 | 0.21 | 0.22 | 2.40 |
| **24** | 6.03 | 0.98 | 0.09 | 0.12 | 3.38 |
| **25** | 5.14 | 1.93 | 0.26 | 0.19 | 1.65 |
| **26** | 4.90 | 1.81 | 0.27 | 0.19 | 1.78 |
| **R44** | 1.46 | 0.42 | 0.13 | 0.77 | 0.23 |
| **R45** | 8.28 | 3.36 | 0.12 | 0.06 | 1.68 |
| **R46** | 2.46 | 0.55 | 0.22 | 0.82 | 0.77 |

Noteworthy is the considerably lower content in anhydrovinblastine (AHVB) in the plants **R44** and **R46.** This is accompanied by an increase in another, as yet unidentified bisindole alkaloid, marked "?". The unknown bisindole is thought to be a close relative of vinblastine since the UV spectra for these two compounds are nearly identical. Possible candidate compounds include **N-demethylvinblastine,** deacetoxyvinblastine, 15'-hydroxyvinblastine or 14'-hydroxyvinblastine.

The content of vinblastine (one of the monomers of the bisindole end products) also varied considerably. These levels seemed to correlate with the ajmalicine contents, but not with those of the bisindole type alkaloids.

**Table 3. Concentrations in µmoles/g DW of some indole alkaloids in the leaf material tested. Results of triplicate determinations shown (n.d. = not detectable, - = not measured).**

| | **Ajmalicine** | **vindoline** | **vinblastine** | **?** | **AHVB** |
|---|---|---|---|---|---|
| **01** | 6.35 | 2.42 | 0.20 | 0.18 | 2.52 |
| | 5.29 | 1.82 | 0.19 | 0.05 | 2.17 |
| | 9.80 | 4.56 | 0.36 | n.d. | 5.12 |
| | | | | | |
| **02** | - | - | - | - | - |
| | 10.69 | 4.53 | 0.39 | 0.17 | 3.77 |
| | 11.34 | 4.69 | 0.31 | 0.14 | 3.16 |
| | | | | | |
| **03** | 4.92 | 1.62 | 0.21 | 0.31 | 2.20 |
| | 5.71 | 1.87 | 0.20 | 0.20 | 2.54 |
| | 6.19 | 1.98 | 0.21 | 0.17 | 2.45 |
| | | | | | |
| **24** | 6.20 | 1.02 | 0.12 | 0.28 | 3.31 |
| | 5.72 | 0.97 | 0.06 | 0.05 | 3.23 |
| | 6.18 | 0.96 | 0.08 | 0.03 | 3.61 |
| | | | | | |
| **25** | 4.69 | 1.69 | 0.25 | 0.19 | 1.59 |
| | 5.13 | 2.01 | 0.30 | 0.17 | 1.94 |
| | 5.61 | 2.10 | 0.23 | 0.22 | 1.40 |
| **26** | 3.77 | 1.49 | 0.28 | 0.21 | 1.56 |
| | 5.69 | 2.11 | 0.30 | 0.15 | 2.16 |
| | 5.24 | 1.85 | 0.24 | 0.21 | 1.62 |
| | | | | | |
| **R44** | 1.56 | 0.37 | 0.15 | 0.96 | 0.12 |
| | 1.52 | 0.49 | 0.15 | 0.70 | 0.46 |
| | 1.31 | 0.41 | 0.10 | 0.67 | 0.11 |
| **R45** | 8.74 | 3.44 | 0.12 | 0.05 | 1.66 |
| | 6.65 | 2.87 | 0.15 | 0.08 | 1.84 |
| | 9.46 | 3.76 | 0.08 | 0.05 | 1.55 |
| | | | | | |
| **R46** | 1.92 | 0.59 | 0.18 | 0.79 | 0.53 |
| | 2.48 | 0.60 | 0.31 | 0.95 | 0.68 |
| | 2.97 | 0.47 | 0.18 | 0.72 | 1.10 |

### Conclusions

Indole alkaloid determination of lyophilized plant leaf preparations showed differing levels of ajmalicine, vindoline, vinblastine, anhydrovinblastine and an as yet unidentified bisindole alkaloid, relative to controls under light treatment regimes as hereindescribed.

### 1. Plant Leaf Material Exposed to Red and Blue Light on Adaxial and Abaxial Leaf Surface

Plants (*Catharanthus roseus*, Chinese cabbage, peas, broccoli, spinach, and *Arabidopsis thaliana*) are grown as described hereinabove and plant leaf material is taken therefrom and subjected to red and blue light in the following ratios (see below) over 2 hours with red light (640 nm +/- 15 nm) being shone onto the upper surface (adaxial surface) and blue light (450 +/- 15nm) being shone onto the under surface (abaxial surface).

Plant material is selected from plants as described above and lyophilised according to standard procedures.

| Blue: Red Light Ratio |
|---|
| 5:1 |
| 5:2 |
| 5:3 |
| 5:4 |
| 5:5 |
| 4:5 |
| 3:5 |
| 2:5 |
| 1:5 |

Treated plants exposed to red and blue light in the above red:blue light ratios are examined as hereindescribed for alterations in plant secondary metabolite concentrations. Alterations in plant secondary metabolite concentrations are observed.

### 1. Plant Leaf Material Exposed to Red and Blue Light on i) the Adaxial Leaf Surface and ii) the Abaxial Leaf Surface

Plants (*Catharanthus roseus*, Chinese cabbage, peas, broccoli, spinach, and *Arabidopsis thaliana*) are grown as described hereinabove and plant leaf material is taken therefrom and subjected to red and blue light in the following ratios (see below) over 2 hours with red light (640 nm +/- 15 nm) and blue light (450 +/- 15nm) being shone onto the upper surface (adaxial surface) of the leaves.

The same methodology is used to shine red and blue light at the same wavelengths on the abaxial surface of the leaves.

Plant material is selected from plants as described above and lyophilised according to standard procedures.

| Blue: Red Light Ratio |
|---|
| 5:1 |
| 5:2 |
| 5:3 |
| 5:4 |
| 5:5 |
| 4:5 |
| 3:5 |
| 2:5 |
| 1:5 |

Treated plants exposed to red and blue light in the above red:blue light ratios are examined as hereindescribed for alterations in plant secondary metabolite concentrations. Alterations in plant secondary metabolite concentrations are observed.

### Apparatus

Referring now to the accompanying drawings (Figure 1), there is shown a schematic elevation of apparatus 10 suitable for performance of a method exemplifying the invention. The apparatus 10 has the form, by way of an example only, of a domestic appliance suitable for kitchen use and comprises a housing 11 of generally cuboidal form with permanently closed ceiling, base and three walls, the fourth wall (not shown) functioning as a door affording access to the interior of the housing.

The housing bounds an exposure chamber which has, in an approximately central position a glass plate 12 serving as a support for plant material 13 to be exposed to treatment light in the chamber. Such light is generated by three mutually separate light sources 14 disposed in the upper region of the chamber and having light exit surfaces 15 oriented to direct light generally towards the top of the plate 12 and thus the upper surface of plant material supported thereon and generally laterally of the plate towards the base of the chamber. Disposed in the vicinity of the base and in such positions as to intercept the laterally directed light are reflectors 16 in the form of mirrors angled so that incident light is directed towards the underside of the plate 12 and thus the lower surface of the plant material, the lower surface being exposed to the light by virtue of the transparency of the plate. The illustrated location of the reflectors 16 and associated reflected light beams is merely by way of example and further such reflectors may be provided to reflect beams obliquely forwardly and backwardly with respect to the plane of the drawing. The material 13 supported on the plate 12 is thus exposed to light at both its upper and lower surface and, to varying degrees, at its side surfaces. Such a disposition of light sources and reflectors has been found to provide a compromise between effective exposure of supported plant material to the generated light and a simple construction with economic operating costs.

The light sources include transmission filters to pass on only light of a selected wavelength or selected wavelengths in the range of 400 to 700 nm and are so controlled by a programmable timer 17 in power feeds 18 to the sources as to emit light for a period of time predetermined to be sufficient to achieve the desired transient alteration in the cell or tissue phytochemicals of the treated plant material.

The appliance is thus conveniently usable for performance of the treatment method immediately prior to cooking or consumption of the treated material.

## Claims

1. A method of transiently altering the level of at least one phytochemical selected from antioxidants in a harvested plant cell comprising chlorophyll or in harvested plant tissue comprising chlorophyll, the plant cell or tissue being capable of photosynthesis, by irradiating the said plant cell or plant tissue with white light enriched with blue light of at least one wavelength selected from the range of wavelengths from 420nm to 490nm to induce or saturate oxygen evolution at the photosystem II reaction centre, wherein the irradiation is carried out over a time interval of up to 180 minutes.

2. A method according to claim 1, wherein the at least one wavelength of light is blue light of a wavelength that lies in the range of from 430nm - 470nm.

3. A method according to claim 1 or claim 2, wherein the wavelength of said blue light lies in the range of from 435nm - 465nm.

4. A method according to any one of the preceding claims, wherein the said white light is further enriched with red light of a wavelength that lies in the range of from 600nm - 700nm.

5. A method according to claim 4, wherein the energy ratio of Blue light:Red light lies in the range of from 7:1 to 1:7.

6. A method according to claim 5, wherein the energy ratio of Blue light:Red light lies in the range of from 6:1 to 1:6.

7. A method according to claim 5 or claim 6, wherein the energy ratio of Blue light:Red light lies in the range of from 5:1 to 1:5.

8. A method according to any one of the preceding claims, wherein the time interval is up to 120 minutes.

9. A method according to claim 8, wherein the time interval is up to 60 minutes.

10. A method according to claim 8 or claim 9, wherein the time interval is up to 45 minutes.

11. A method according to any one of claims 8 to claim 10, wherein the time interval is up to 30 minutes.

12. A method according to any one of claims 8 to 11, wherein the time interval lies in the range of from 5 to 15 minutes.

13. A method according to any one of the preceding claims, wherein the plant cell or plant tissue is selected from plant tissue capable of photosynthesis that is selected from green stems, calyx, and leaves of higher order plants, algal cells, moss protonema and cell cultures of edible and/or inedible or unpalatable higher and lower plant species.

14. A method according to claim 13, wherein the plant cell or plant tissue is obtained from a plant selected from the group comprising herbs, *Catharanthus roseus,* plants of the family Taxaceae, Cannabis plants, green vegetables and green seeds.

15. A method according to claim 13 or claim 14, wherein the plant cell or plant tissue is obtained from a plant selected from the group comprising *Catharanthus roseus,* peas, green beans, spinach, species from the *Brassica oleracea* such as broccoli, green cabbage, red cabbage, Brussels sprouts, kohlrabi, cauliflower, white cabbage, lettuce, Chinese cabbage, moss tissue such as protonema of *Physcomitrella patens*, cultures of lemnospora species, and algal cell cultures.

16. A method according to any one of the preceding claims, wherein the phytochemical is selected from Vitamin C and vitamin E.

## Patentansprüche

1. Verfahren für die vorübergehende Änderung des Gehalts an mindestens einer phytochemischen Verbindung aus der Reihe der Antioxidantien in einer geernteten chlorophyllhaltigen Pflanzenzelle oder in geerntetem chlorophyllhaltigem Pflanzengewebe, wobei die Pflanzenzelle bzw. das Pflanzengewebe zur Photosynthese befähigt ist, durch Bestrahlen der Pflanzenzelle bzw. des Pflanzengewebes mit Weißlicht, das mit Blaulicht mit mindestens einer Wellenlänge ausgewählt aus der Reihe der Wellenlängen von 420 nm bis 490 nm angereichert ist, zur Induktion oder Sättigung der Entstehung von Sauerstoff am Reaktionszentrum des Photosystems II, wobei die Bestrahlung über einen Zeitraum von bis zu 180 Minuten erfolgt.

2. Verfahren nach Anspruch 1, wobei es sich bei der mindestens einen Wellenlänge des Lichts um Blaulicht mit einer Wellenlänge, die im Bereich von 430 nm - 470 nm liegt, handelt.

3. Verfahren nach Anspruch 1 oder Anspruch 2, wobei die Wellenlänge des Blaulichts im Bereich von 435 nm - 465 nm liegt.

4. Verfahren nach einem der vorhergehenden Ansprüche, wobei das Weißlicht weiterhin mit Rotlicht einer Wellenlänge, die im Bereich von 600 nm - 700 nm liegt, angereichert ist.

5. Verfahren nach Anspruch 4, wobei das Energieverhältnis von Blaulicht zu Rotlicht im Bereich von 7:1 bis 1:7 liegt.

6. Verfahren nach Anspruch 5, wobei das Energieverhältnis von Blaulicht zu Rotlicht im Bereich von 6:1 bis 1:6 liegt.

7. Verfahren nach Anspruch 5 oder Anspruch 6, wobei das Energieverhältnis von Blaulicht zu Rotlicht im Bereich von 5:1 bis 1:5 liegt.

8. Verfahren nach einem der vorhergehenden Ansprüche, wobei der Zeitraum bis zu 120 Minuten beträgt.

9. Verfahren nach Anspruch 8, wobei der Zeitraum bis zu 60 Minuten beträgt.

10. Verfahren nach Anspruch 8 oder Anspruch 9, wobei der Zeitraum bis zu 45 Minuten beträgt.

11. Verfahren nach einem der Ansprüche 8 bis 10, wobei der Zeitraum bis zu 30 Minuten beträgt.

12. Verfahren nach einem der Ansprüche 8 bis 11, wobei der Zeitraum im Bereich von 5 bis 15 Minuten liegt.

13. Verfahren nach einem der vorhergehenden Ansprüche, wobei die Pflanzenzelle oder das Pflanzengewebe aus zur Photosynthese befähigtem Pflanzengewebe, ausgewählt aus der Reihe grüne Stengel, Kelch sowie Blätter von höheren Pflanzen, Algenzellen, Moos-Protonema und Zellkulturen von eßbaren und/oder nichteßbaren oder ungenießbaren Arten von höheren und niedrigeren Pflanzen, ausgewählt ist.

14. Verfahren nach Anspruch 13, wobei die Pflanzenzelle bzw. das Pflanzengewebe von einer Pflanze, ausgewählt aus der Gruppe umfassend Kräuter, *Catharanthus roseus,* Pflanzen der Familie Taxaceae, Cannabis-Pflanzen, grünes Gemüse und grünen Samen, stammt.

15. Verfahren nach Anspruch 13 oder Anspruch 14, wobei die Pflanzenzelle bzw. das Pflanzengewebe von einer Pflanze ausgewählt aus der Gruppe umfassend *Catharanthus roseus,* Erbsen, grüne Bohnen, Spinat, *Brassicaoleracea*-Arten wie Brokkoli, Grünkohl, Rotkohl, Rosenkohl, Kohlrabi, Blumenkohl, Weißkohl, Salat, Chinakohl, Moosgewebe wie Protonema von *Physcomitrella patens,* Kulturen von Lemnospora-Arten und Algenzellkulturen, stammt.

16. Verfahren nach einem der vorhergehenden Ansprüche, wobei die phytochemische Verbindung aus der Reihe Vitamin C und Vitamin E ausgewählt ist.

## Revendications

1. Procédé de modification transitoire d'au moins un composé phytochimique choisi parmi les antioxydants dans une cellule végétale récoltée comprenant de la chlorophylle ou dans un tissu végétal récolté comprenant de la chlorophylle, la cellule ou le tissu végétal étant capable de photosynthèse, par irradiation de ladite cellule végétale ou dudit tissu végétal avec une lumière blanche enrichie par une lumière bleue d'au moins une longueur d'onde choisie dans la gamme de longueurs d'onde de 420 nm à 490 nm pour induire ou saturer le dégagement d'oxygène au centre réactionnel du photosystème II, dans lequel l'irradiation est réalisée sur un intervalle de temps allant jusqu'à 180 minutes.

2. Procédé selon la revendication 1, dans lequel l'au moins une longueur d'onde de lumière est une lumière bleue d'une longueur d'onde qui se situe dans la gamme de 430 nm à 470 nm.

3. Procédé selon la revendication 1 ou la revendication 2, dans lequel la longueur d'onde de ladite lumière bleue se situe dans la gamme de 435 nm à 465 nm.

4. Procédé selon l'une quelconque des revendications précédentes, dans lequel ladite lumière blanche est en outre enrichie par une lumière rouge d'une longueur d'onde qui se situe dans la gamme de 600 nm à 700 nm.

5. Procédé selon la revendication 4, dans lequel le rapport énergétique lumière bleue/lumière rouge se situe dans la gamme de 7/1 à 1/7.

6. Procédé selon la revendication 5, dans lequel le rapport énergétique lumière bleue/lumière rouge se situe dans la gamme de 6/1 à 1/6.

7. Procédé selon la revendication 5 ou la revendication 6, dans lequel le rapport énergétique lumière bleue/lumière rouge se situe dans la gamme de 5/1 à 1/5.

8. Procédé selon l'une quelconque des revendications précédentes, dans lequel l'intervalle de temps va jusqu'à 120 minutes.

9. Procédé selon la revendication 8, dans lequel l'intervalle de temps va jusqu'à 60 minutes.

10. Procédé selon la revendication 8 ou la revendication 9, dans lequel l'intervalle de temps va jusqu'à 45 minutes.

11. Procédé selon l'une quelconque des revendications 8 à 10, dans lequel l'intervalle de temps va jusqu'à 30 minutes.

12. Procédé selon l'une quelconque des revendications 8 à 11, dans lequel l'intervalle de temps se situe dans la gamme de 5 à 15 minutes.

13. Procédé selon l'une quelconque des revendications précédentes, dans lequel la cellule végétale ou le tissu végétal est sélectionné à partir d'un tissu végétal capable de photosynthèse qui est choisi parmi les tiges vertes, le calice et les feuilles des plantes d'ordre supérieur, les cellules algales, le protonéma des mousses, et les cultures cellulaires d'espèces végétales supérieures et inférieures comestibles et/ou non comestibles ou inappétentes.

14. Procédé selon la revendication 13, dans lequel la cellule végétale ou le tissu végétal est obtenu à partir d'une plante choisie dans le groupe comprenant les herbes, *Catharanthus roseus,* les plantes de la famille des taxacées, les plantes de cannabis, les légumes verts, et les graines vertes.

15. Procédé selon la revendication 13 ou la revendication 14, dans lequel la cellule végétale ou le tissu végétal est obtenu à partir d'une plante choisie dans le groupe comprenant *Catharanthus roseus,* les pois, les haricots verts, les épinards, les espèces de *Brassica oleracea* telles que le brocoli, le chou vert, le chou rouge, le chou de Bruxelles, le chou-rave, le chou-fleur, le chou blanc, la laitue, le chou chinois, les tissus de mousses tels que le protonéma de *Physcomitrella patens*, les cultures d'espèces de *lemnospora*, et les cultures de cellules algales.

16. Procédé selon l'une quelconque des revendications précédentes, dans lequel le composé phytochimique est choisi parmi la vitamine C et la vitamine E.
